# EUROPEAN PATENT SPECIFICATION

(11) **EP 3 747 477 B1**
(45) Date of publication and mention of the grant of the patent: **16.11.2022**
(21) Application number: 18903638.7
(22) Date of filing: 23.08.2018
(51) Int. Cl.: A61L 27/38, A61L 27/36, A61L 27/22, A61L 27/54, A61F 2/30, B33Y 80/00

(54) **BIOINK COMPOSITION FOR CARTILAGE REGENERATION, METHOD FOR MANUFACTURING CUSTOMIZED SCAFFOLD FOR CARTILAGE REGENERATION USING SAME, AND CUSTOMIZED SCAFFOLD FOR CARTILAGE REGENERATION MANUFACTURED USING MANUFACTURING METHOD**
BIOTINTENZUSAMMENSETZUNG FÜR DIE KNORPELREGENERATION, VERFAHREN ZUR HERSTELLUNG EINES KUNDENSPEZIFISCHEN GERÜSTES FÜR DIE KNORPELREGENERATION DAMIT UND UNTER VERWENDUNG DES HERSTELLUNGSVERFAHRENS HERGESTELLTES KUNDENSPEZIFISCHES GERÜST FÜR DIE KNORPELREGENERATION
COMPOSITION DE BIOENCRE POUR LA RÉGÉNÉRATION DU CARTILAGE, PROCÉDÉ DE FABRICATION D'UN ÉCHAFAUDAGE PERSONNALISÉ POUR LA RÉGÉNÉRATION DU CARTILAGE FAISANT APPEL À CELLE-CI, ET ÉCHAFAUDAGE PERSONNALISÉ POUR LA RÉGÉNÉRATION DU CARTILAGE FABRIQUÉ EN FAISANT APPEL AU PROCÉDÉ DE FABRICATION

(30) Priority: 31.01.2018 KR 20180012221
(43) Date of publication of application: 09.12.2020
(73) Proprietor: Rokit Healthcare Inc., Geumcheon-gu Seoul 08512 (KR)
(72) Inventor: YOU, Seok Hwan, Seoul 06943 (KR)
(74) Representative: Maiwald Patent- und Rechtsanwaltsgesellschaft mbH
(86) International application number: PCT/KR2018/009707
(87) International publication number: WO 2019/151597

(56) References cited:
- EP-A1- 2 998 392
- EP-A2- 0 339 607
- WO-A1-2015/048317
- WO-A1-2015/048317
- KR-A- 20100 041 027
- KR-A- 20130 037 324
- US-A1- 2016 095 958
- AM MÜLLER ET AL: "Towards an intraoperative engineering of osteogenic and vasculogenic grafts from the stromal vascular fraction of human adipose tissue", EUROPEAN CELLS AND MATERIALS, vol. 19, 1 January 2010 (2010-01-01), pages 127-135, XP055628596, DOI: 10.22203/eCM.v019a13
- SEUNGBUM KOO ET AL: "Fabrication of custom-shaped grafts for cartilage regeneration", INTERNATIONAL JOURNAL OF ARTIFICIAL ORGANS, vol. 33, no. 10, 1 January 2010 (2010-01-01), pages 731-737, XP055843186,
- MULLER, A.M.et al.: "Towards an intraoperative engineering of osteogenic and vasculogenic grafts from the stromal vascular fraction of human adipose tissue", European Cells and Materials, vol. 19, 2010, pages 127-135, XP055628596,

## Description

### Technical Field

The present specification claims priority to and the benefit of Korean Patent Application No. 10-2018-0012221 filed in the Korean Intellectual Property Office on January 31, 2018.

The present invention relates to a bioink composition for cartilage regeneration, a method for manufacturing a customized scaffold for cartilage regeneration using the same, and a customized scaffold for cartilage regeneration manufactured using said manufacturing method.

### Background Art

Degenerative arthritis is a disease in which local degenerative changes appear while the articular cartilage wears away, and is also called osteoarthritis or osteoarthrosis. Degenerative arthritis is one type of chronic arthritis, and is a disease in which a degenerative change occurs in the articular cartilage that receives a weight load, resulting in overgrowth of bone on the joint surface, and is often seen in middle-aged adults. In this disease, first, cartilage loses elasticity while the function of chondrocytes which produce cartilage composition components deteriorates due to aging. Moreover, as time passes, the surface of the cartilage becomes rough, and various types of materials flow into the joint cavity surrounded by the joint membrane to cause inflammation. Clinically, recurrent pain, joint stiffness, a progressive movement disorder in the joint, and the like appear. The cause of degenerative arthritis is closely associated with aging phenomena or excessive body weight, and degenerative arthritis more frequently and more seriously appears in women as they get older. As an initial symptom, one or two joints exhibit a throbbing pain along with stiffness, and when prolonged, the hardening of the subchondral bone, excessive formation of the bone around the joint, deformation of the joint, and the like are caused.

The cause of degenerative changes in articular cartilage has not yet been elucidated, but the absolute decrease in the number of chondrocytes and the imbalance between synthesis and degradation of the cartilage matrix occurring in chondrocytes are known to be one of the causes. Therefore, degenerative changes in articular cartilage reduce cartilage strength and the ability as a cushion due to the degradation of the cartilage matrix.

Existing cell therapeutic agents have a disadvantage in that implanted cells are biased in one direction due to gravity, so that it is difficult to evenly distribute the therapeutic agent in a defect site, and there is difficulty in cell engraftment, which reduces cell regeneration in the cartilage defect site. Further, in the case of autologous chondrocyte implantation and stem cell implantation, a tissue is applied to a patient through a culture process after the tissue is extracted, so that two surgeries are required, and an approximate 4-week cell culture period or manipulation is required. In addition, there are problems in that due to gravity, it is difficult for implanted cells to be evenly distributed and engrafted in a defect site, and the differentiation into the fibrocartilage instead of the hyaline cartilage is induced, so that a phenomenon in which the cartilage breaks down easily occurs. Therefore, there is a need for developing a method capable of effectively regenerating cells in a cartilage defect site.

### Prior Art Documents

### Patent Document

KR Patent Application Laid-Open No. 10-2017-0012099

EP2998392 discloses the preparation of fibrin hydrogel / cartilage scaffolds comprising fat pad derived stromal cells by mixing cryomilled hyaline cartilage particles (1.5% w/v) with fibrinogen (100 mg/ml), mixing the suspension with the stromal cells, adding to appropriate shaped molds, followed by adding thrombin.

### Disclosure

### Technical Problem

The present invention has been made in an effort to provide a bioink composition for cartilage regeneration for manufacturing a customized scaffold for cartilage regeneration of a defect cartilage site. Furthermore, the present invention has been made in an effort to provide a method for manufacturing a customized scaffold for cartilage regeneration using the bioink composition for cartilage regeneration, and a customized scaffold for cartilage regeneration manufactured using the same.

### Technical Solution

The present invention provides a bioink composition for cartilage regeneration, the bioink composition comprising: a first liquid comprising an adipose tissue-derived stromal vascular fraction, hyaline cartilage powder, and fibrinogen, wherein the first liquid comprises fibrinogen in an amount of 65 mg to 115 mg per ml of the first liquid; and a second liquid comprising thrombin dissolved in a calcium chloride solution, wherein the second liquid comprises thrombin in an amount of 400 IU to 600 IU and 5 mg to 6.5 mg of calcium chloride per ml of the second liquid.

The present invention provides a method for manufacturing a customized scaffold for cartilage regeneration, the method comprising: a) obtaining 3D data of a defect cartilage site using a 3D scanner, and manufacturing a mold for scaffolding using a 3D printer; b) using a first liquid comprising an adipose tissue-derived stromal vascular fraction, hyaline cartilage powder, and fibrinogen, wherein the first liquid comprises fibrinogen in an amount of 65 mg to 115 mg per ml of the first liquid, and applying the first liquid in the mold for scaffolding to form a first layer; c) applying a second liquid comprising thrombin dissolved in a calcium chloride solution onto the first layer to form a second layer, wherein the second liquid comprises thrombin in an amount of 400 IU to 600 IU and 5 mg to 6.5 mg of calcium chloride per ml of the second liquid; and d) forming a customized scaffold for cartilage regeneration by allowing the first layer and the second layer to react with each other.

The present invention provides a customized scaffold for cartilage regeneration manufactured using the manufacturing method.

### Advantageous Effects

A bioink composition for cartilage regeneration according to the present invention has an advantage in that a patient-customized scaffold for cartilage regeneration can be manufactured.

A customized scaffold for cartilage regeneration manufactured using the bioink composition for cartilage regeneration according to the present invention can effectively treat the cartilage by enhancing the ability of a implanted adipose tissue-derived stromal vascular fraction to differentiate into cartilage, and furthermore, increasing regeneration of cartilage in the implanted site.

The customized scaffold for cartilage regeneration according to the present invention has an excellent bonding force to an affected area, and has an advantage in that cartilage differentiation ability and a cartilage regeneration effect are excellent due to the excellent bonding force.

A method for manufacturing a customized scaffold for cartilage regeneration according to the present invention has an advantage in that a scaffold can be implanted through a single surgical procedure because a customized scaffold for cartilage regeneration can be immediately manufactured by 3D-scanning a cartilage defect site of an affected area in an operation room.

### Description of Drawings

FIG. 1 illustrates a process of manufacturing a pellet for confirming the cartilage differentiation ability of Experimental Example 1.
FIG. 2 shows a result of performing safranin O staining on a pellet of Comparative Example 1 (MCCM 0 mg) according to Experimental Example 1.
FIG. 3 shows a result of performing safranin O staining on a pellet of Example 1 (MCCM 10 mg) according to Experimental Example 1.
FIG. 4 shows a result of performing safranin O staining on a pellet of Example 2 (MCCM 50 mg) according to Experimental Example 1.
FIG. 5 shows a result of performing safranin O staining on a pellet of Example 3 (MCCM 100 mg) according to Experimental Example 1.
FIG. 6 illustrates an animal experimental procedure for confirming whether the cartilage of Experimental Example 2 is regenerated.
FIG. 7 shows images during an animal experimental procedure of Example 4 according to Experimental Example 2 and a micro CT imaging image for a defect region 6 weeks after the implantation of a scaffold.
FIG. 8 shows images during an animal experimental procedure of Comparative Example 2 according to Experimental Example 2 and a micro CT imaging image for a defect region 6 weeks after the implantation of a scaffold.
FIG 9 shows a result of performing safranin O staining on a defect region 6 weeks after the implantation of Comparative Example 2 according to Experimental Example 2.
FIG. 10 shows a result of performing safranin O staining on a defect region 6 weeks after the implantation of Example 4 according to Experimental Example 2.
FIG. 11 shows a result of performing an immunohistochemistry (IHC) treatment with collagen type 1 on a defect region 6 weeks after the implantation of Comparative Example 2 according to Experimental Example 2.
FIG. 12 shows a result of performing an immunohistochemistry (IHC) treatment with collagen type 1 on a defect region 6 weeks after the implantation of Example 4 according to Experimental Example 2.
FIG. 13 shows a result of performing an immunohistochemistry (IHC) treatment with collagen type 2 on a defect region 6 weeks after the implantation of Comparative Example 2 according to Experimental Example 2.
FIG. 14 shows a result of performing an immunohistochemistry (IHC) treatment with collagen type 2 on a defect region 6 weeks after the implantation of Example 4 according to Experimental Example 2.

### Modes of the Invention

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

Hereinafter, the present invention will be described in detail.

The present invention provides a bioink composition for cartilage regeneration, the bioink composition comprising: a first liquid comprising an adipose tissue-derived stromal vascular fraction, hyaline cartilage powder, and fibrinogen, wherein the first liquid comprises fibrinogen in an amount of 65 mg to 115 mg per ml of the first liquid; and a second liquid comprising thrombin dissolved in a calcium chloride solution, wherein the second liquid comprises thrombin in an amount of 400 IU to 600 IU and 5 mg to 6.5 mg of calcium chloride per ml of the second liquid.

The bioink composition for cartilage regeneration according to the present invention is a two-liquid type, and the first liquid and the second liquid are sequentially applied and then reacted to form a scaffold for cartilage regeneration. Specifically, the thrombin in the second liquid may be reacted with the fibrinogen in the first liquid to form a fibrin network, which may serve to sufficiently fix the adipose tissue-derived stromal vascular fraction and the hyaline cartilage powder.

The adipose tissue-derived stromal vascular fraction includes adipose derived stem cells. Preferably, the adipose tissue-derived stromal vascular fraction may not substantially include cells (for example, adipocytes, erythrocytes, other stromal cells, and the like) other than adipose tissue-derived stem cells and extracellular matrix materials, and more preferably, may not include other cells and extracellular matrix materials at all.

The adipose tissue-derived stromal vascular fraction may be extracted from an adipose tissue of an allogeneic animal or a heterologous animal. Preferably, the adipose tissue-derived stromal vascular fraction may be extracted from an autologous adipose tissue. More specifically, the adipose tissue-derived stromal vascular fraction may be extracted using adipocytes of a patient or animal to be treated.

According to an exemplary embodiment of the present invention, a content of the adipose tissue-derived stromal vascular fraction may be 10⁵ to 10⁷ cells/ml of the first solution. When the content of the adipose tissue-derived stromal vascular fraction is within the above range, the cartilage differentiation ability and cartilage regeneration ability of a scaffold to be manufactured may be significantly improved.

The adipose tissue-derived stromal vascular fraction may be used with hyaline cartilage powder to differentiate into cartilage calls, and when the customized scaffold for cartilage regeneration is implanted into an affected area for this reason, the differentiation into chondrocytes may be actively induced.

The hyaline cartilage powder may be an element which constitutes a scaffold support manufactured using the bioink composition for cartilage regeneration. Specifically, a fibrin matrix through the reaction of fibrinogen and thrombin may allow particles of the hyaline cartilage powder to be bonded to one another.

The hyaline cartilage powder may be derived from allogeneic or heterologous hyaline cartilage. Preferably, the hyaline cartilage powder may be derived from allogeneic hyaline cartilage. Specifically, when a subject to undergo cartilage treatment is a human, hyaline cartilage powder extracted from human hyaline cartilage may be used. Further, when a subject to undergo cartilage treatment is an animal, hyaline cartilage powder extracted from hyaline cartilage of an animal of the same species may be used.

The hyaline cartilage powder may induce differentiation of the adipose tissue-derived stromal vascular fraction into chondrocytes by releasing cartilage regeneration-related growth factors. Furthermore, the proteins included in the hyaline cartilage powder may serve to aid in active regeneration of cartilage in an affected area.

According to an exemplary embodiment of the present invention, the hyaline cartilage powder may be derived from costal cartilage. Specifically, when the customized scaffold for cartilage regeneration is applied to a human, the hyaline cartilage powder may be derived from human costal cartilage. Further, when the customized scaffold for cartilage regeneration is applied to an animal, the hyaline cartilage powder may be derived from the costal cartilage of the same species as the subject animal. For example, the hyaline cartilage powder may be used by powdering commercially available allogeneic costal cartilage.

According to an exemplary embodiment of the present invention, the hyaline cartilage powder may have a particle diameter of 30 µm to 300 µm. When the average particle diameter of the hyaline cartilage powder is within the above range, there is an advantage in that chondrocytes are effectively differentiated and regenerated.

According to an exemplary embodiment of the present invention, the concentration of the hyaline cartilage powder may be 0.005 %(w/v) to 0.1 %(w/v). That is, the hyaline cartilage powder may be included in an amount of 0.005 g to 0.1 g per ml of the first liquid. A concentration of the hyaline cartilage powder may be preferably 0.005 %(w/v) to 0.07 %(w/v), more preferably 0.005 %(w/v) to 0.03 %(w/v), and most preferably 0.007 %(w/v) to 0.03 %(w/v). When the concentration of the hyaline cartilage powder is within the above range, the survivability of chondrocytes, differentiation into chondrocytes, and morphology may be most excellently realized after implantation of a customized scaffold for cartilage regeneration.

According to an exemplary embodiment of the present invention, the cell number of the adipose tissue-derived stromal vascular fraction may be 10⁵ to 10⁷ with respect to 5 mg to 100 mg of the hyaline cartilage powder. Preferably, the cell number of the adipose tissue-derived stromal vascular fraction may be 10⁵ to 10⁷ with respect to 5 mg to 70 mg of the hyaline cartilage powder. More preferably, the cell number of the adipose tissue-derived stromal vascular fraction may be 10⁵ to 10⁷ with respect to 5 mg to 30 mg of the hyaline cartilage powder.

According to an exemplary embodiment of the present invention, the first liquid may further include aprotinin. The aprotinin is an inhibitor of proteolytic enzymes secreted from the pancreas, and is a polypeptide consisting of a total of 58 amino acids. The aprotinin is usually extracted from the lungs of cattle, and is known to perform a hemostatic action by inhibiting the degradation of fibrin in blood.

According to the present invention, the fibrinogen is included in an amount of 65 mg to 115 mg per ml of the first liquid.

According to an exemplary embodiment of the present invention, the aprotinin may be included in an amount of 900 kininogen inactivator units (KIU) to 1,1000 KIU, specifically 1,000 KIU, per ml of the first liquid.

Specifically, according to an exemplary embodiment of the present invention, the first liquid may include 10⁵ to 10⁷ adipose tissue-derived stromal vascular fraction cells, 5 mg to 100 mg of hyaline cartilage powder, 65 mg to 115 mg of fibrinogen, and 900 KIU to 1,100 KIU of aprotinin per ml of the first liquid.

According to the present invention, the second liquid comprises thrombin dissolved in a calcium chloride solution. The second liquid includes 400 IU to 600 IU of thrombin and 5 mg to 6.5 mg of calcium chloride per ml of the second liquid.

A solvent of the first liquid and the second liquid may be water, specifically physiological saline. Further, the fibrinogen in the first liquid and the thrombin in the second liquid may be obtained by a commercially available fibrin glue kit.

Since the reaction of the first liquid and the second liquid is completed within 10 minutes, preferably within 5 minutes, the bioink composition for cartilage regeneration has an advantage in that a patient-customized scaffold for cartilage regeneration may be immediately manufactured using a 3D printer at a treatment site.

The present invention uses a fibrin glue including fibrinogen and fibrin as an adhesive, which may secure higher viscosity than that of a hyaluronic acid adhesive or collagen adhesive, so that the customized scaffold for cartilage regeneration has excellent bonding force to an affected area, and furthermore, may maintain high strength.

The present invention provides a method for manufacturing a customized scaffold for cartilage regeneration, the method comprising: a) obtaining 3D data of a defect cartilage site using a 3D scanner, and manufacturing a mold for scaffolding using a 3D printer; b) using a first liquid comprising an adipose tissue-derived stromal vascular fraction, hyaline cartilage powder, and fibrinogen, wherein the first liquid comprises fibrinogen in an amount of 65 mg to 115 mg per ml of the first liquid, and applying the first liquid in the mold for scaffolding to form a first layer; c) applying a second liquid comprising thrombin dissolved in a calcium chloride solution onto the first layer to form a second layer, wherein the second liquid comprises thrombin in an amount of 400 IU to 600 IU and 5 mg to 6.5 mg of calcium chloride per ml of the second liquid; and d) forming a customized scaffold for cartilage regeneration by allowing the first layer and the second layer to react with each other.

In step a), 3D scanner equipment and 3D printing equipment known in the art may be used. Further, the mold for scaffolding may serve to fix a three-dimensional form when the first liquid and the second liquid are applied. The mold for scaffolding may be removed after the scaffold for cartilage regeneration is formed. The mold for scaffolding may be formed using a biocompatible polymer generally used in the art.

According to an exemplary embodiment of the present invention, steps b) and c) may be performed using inkjet printing or 3D printing. Specifically, in steps b) and c), a printing device having two or more nozzles known in the art may be used, and a three-dimensional shape may be created by discharging the first liquid and the second liquid from the respective nozzles.

According to an exemplary embodiment of the present invention, steps b) and c) may be alternately repeated. Specifically, when it is necessary to form a large-volume scaffold for cartilage regeneration, steps b) and c) are alternately performed to laminate layers as in "first layer/second layer/first layer/second layer, and then the layers may be coagulated to form a scaffold for cartilage regeneration.

According to an exemplary embodiment of the present invention, step d) may be completed within 10 minutes, preferably within 5 minutes. Specifically, in step d), a reaction may be performed for 3 minutes to 7 minutes and the first layer and the second layer may be reacted to form a scaffold for cartilage regeneration.

In the case of existing autologous chondrocyte implantation and stem cell implantation, there is a problem in that after the primary incision is made on an affected area to extract a tissue of a patient's defect cartilage area, the secondary incision for implantation to the affected area through a culture process of the tissue is required, so that two surgical procedures are required. In contrast, the method for manufacturing a customized scaffold for cartilage regeneration has an advantage in that after a defect cartilage region is confirmed through the primary incision, a scaffold corresponding to the defect cartilage region is manufactured within a short period of time and may be implanted into the defect cartilage region. That is, the method for manufacturing a customized scaffold for cartilage regeneration has an advantage in that a customized scaffold can be implanted into an affected area by only one incision.

The present invention provides a customized scaffold for cartilage regeneration manufactured using the manufacturing method.

The customized scaffold for cartilage regeneration may be manufactured in a shape corresponding to a defect cartilage region within a short period of time and implanted, as described above. The customized scaffold for cartilage regeneration does not require a culture process for cells, an adipose tissue-derived stromal vascular fraction in the scaffold after implantation differentiates into chondrocytes, and hyaline cartilage powder may activate regeneration of the cartilage and enhance the viability of regenerated chondrocytes.

When the customized scaffold for cartilage regeneration is implanted into an affected area, natural hyaline cartilage may be induced to be regenerated by hyaline cartilage growth factors and proteins secreted from the hyaline cartilage powder. When the customized scaffold for cartilage regeneration is applied to an affected area in this manner, the affected area may be effectively restored to the same or similar state as that of the original cartilage.

Hereinafter, the present invention will be described in detail with reference to Examples for specifically describing the present invention. However, the Examples according to the present invention may be modified into various different forms, and it should not be interpreted that the scope of the present invention is limited to the Examples to be described below. The Examples of the present specification are provided for more completely explaining the present invention to those with ordinary skill in the art.

### Isolation of adipose tissue-derived stromal vascular fraction (SVF) cells

Adipose tissue-derived stromal vascular fraction (SVF) cells were obtained through the following steps.
1. Approximately 60 cc of adipose tissue was extracted by performing liposuction led by a doctor in a sterile operating room, and 0.075% collagenase lipid phage was added in the same amount to the tissue, and the resulting mixture was allowed to react by shaking incubation at 230 rpm at a temperature of 37°C for 30 minutes.
2. After the reaction as described above, 420 g of the reaction product was centrifuged at 25°C for 10 minutes, and as a result of the centrifugation, the reaction product was divided into three layers of an SVF pellet layer, a collagenase layer, and an oil layer.
3. After the upper solution except for the SVF pellet layer was removed and the SVF pellet layer was resuspended in phosphate buffered saline (PBS), fibrous materials and the remaining impurities of the resuspended SVF layer were removed using a 100 µm nylon filter (cell strainer).
4. After a solution including the filtered SVF was resuspended and centrifuged three times, all remaining materials were removed except for the pellet at the bottom layer, and then as a result of calculating nucleated cells using a cell counter, it could be confirmed that 10⁵ to 10⁷ SVF cells were obtained.

### Preparation of hyaline cartilage powder

Hyaline cartilage powder was prepared as a powder having a particle size of 30 µm to 300 µm using commercially available costal cartilage. Hereinafter, the hyaline cartilage powder will be referred to as a micronized costal cartilage matrix.

### Experimental Example 1 - Manufacture of pellet for confirmation of chondrogenic differentiation (in vitro experiment)

### Examples 1 to 3

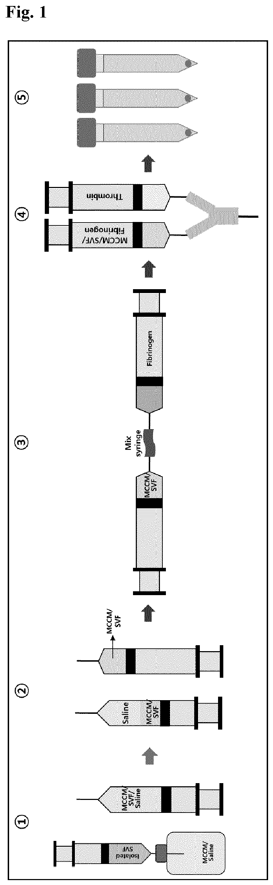

FIG. 1 illustrates a process of manufacturing a pellet for confirming cartilage differentiation ability. Specifically, an experiment for confirming cartilage differentiation ability was performed as follows.
1. The obtained SVF (10⁵ to 10⁷ cells) was mixed with physiological saline in which 10 mg (Example 1), 50 mg (Example 2), and 100 mg (Example 3) of the prepared hyaline cartilage powder (MCCM) were dispersed.
2. After this mixed liquid was centrifuged at 1000 rpm at 4°C for 5 minutes, an MCCM/SVF mixture was obtained by removing the physiological saline supernatant.
3. The MCCM/SVF mixture was mixed with 1 ml of an aprotinin liquid in which fibrinogen was mixed using a mix syringe.
4. 1 ml of the MCCM/SVF/fibrinogen solution and 1 ml of a calcium chloride solution in which thrombin was dispersed were each connected to a Y piece, and 20 µl of each solution was dispensed into a 15-ml conical tube and hardened at room temperature for 5 minutes to form a pellet.
5. Growth factor free media were exchanged and administered three times a week such that the pellet was sufficiently immersed, the pellet was incubated under the conditions of 37°C and 5% CO₂ for 5 weeks, and then chondrogenic differentiation was confirmed by performing safranin O staining.

### Comparative Example 1

A pellet was formed and incubated in the same manner as described above by mixing only SVF with an aprotinin liquid in which fibrinogen was mixed without MCCM (0 mg of MCCM, Comparative Example 1), and then chondrogenic differentiation was confirmed by performing safranin O staining.

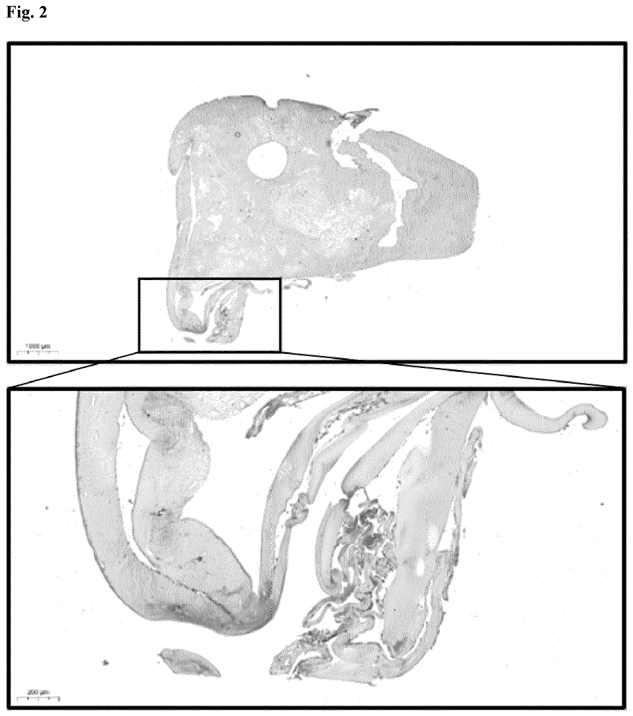

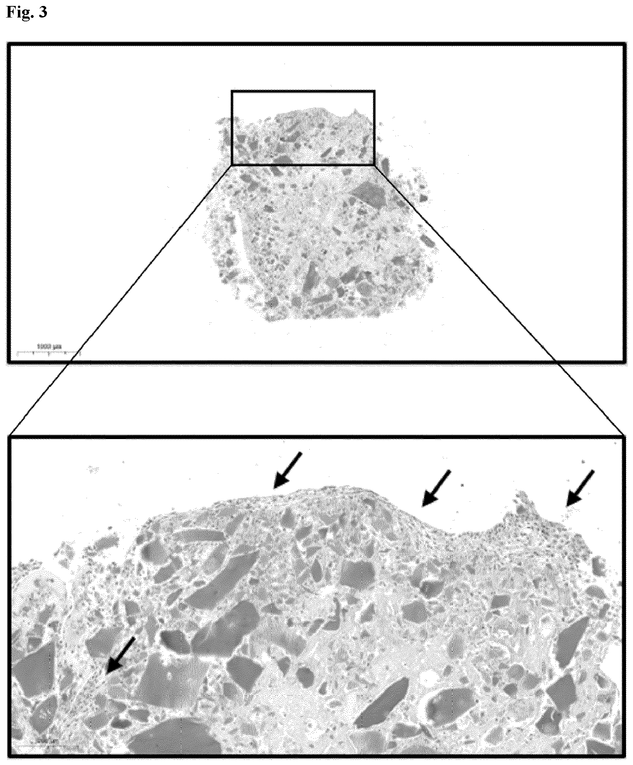

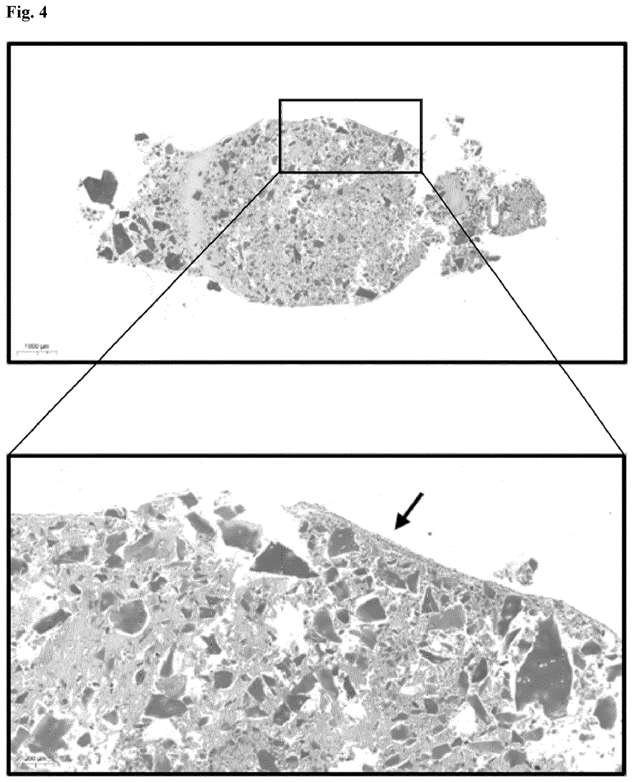

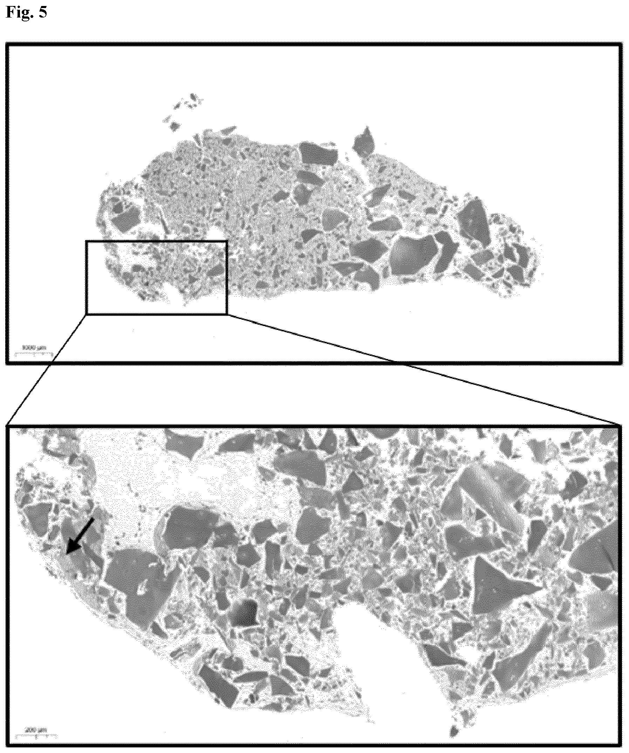

FIGS. 2 to 5 each show a result of performing safranin O staining on a pellet according to Comparative Example 1 (0 mg of MCCM), Example 1 (10 mg of MCCM), Example 2 (50 mg of MCCM), and Example 3 (100 mg of MCCM). In FIGS. 2 to 5, it could be confirmed that the cartilage portion and MCCM were stained red according to safranin O staining and nuclei of chondrocytes were observed around the MCCMs in Examples 1 to 3, and it could be confirmed that a number of living chondrocytes were present. However, it could not be confirmed that in the case of Comparative Example 1 in which MCCM was not used, chondrocytes were differentiated. Through the results, it can be inferred that MCCM proteins, growth factors and the like were released and induced differentiation of SVF into chondrocytes. Furthermore, with respect to the content of MCCM, it could be confirmed that Example 1 (10 mg of MCCM) had the most active chondrocyte viability and differentiation into chondrocytes compared to Examples 2 and 3. That is, when the content of MCCM is too high, it is determined that the differentiation into chondrocytes is delayed because the space where SVF may differentiate into chondrocytes is reduced.

### Experimental Example 2 - Animal experiment for confirmation of cartilage regeneration (in vivo experiment)

### Example 4

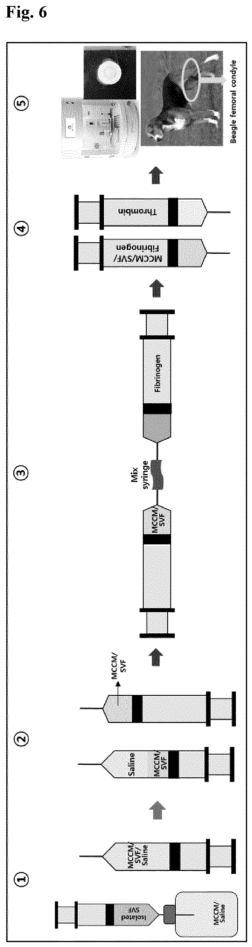

FIG. 6 illustrates an animal experimental procedure for confirming whether the cartilage is regenerated. Specifically, an animal experiment for confirming whether the cartilage was regenerated was performed as follows.
1. The obtained SVF (10⁵ to 10⁷ cells) were mixed with physiological saline in which 10 mg of the prepared hyaline cartilage powder (MCCM) was dispersed.
2. After this mixed liquid was centrifuged at 1000 rpm at 4°C for 5 minutes, an MCCM/SVF mixture was obtained by removing the physiological saline supernatant.
3. The MCCM/SVF mixture was mixed using 1 ml of an aprotinin solution in which fibrinogen was mixed and a mix syringe.
4. 1 ml of the MCCM/SVF/fibrinogen solution and 1 ml of a calcium chloride solution in which thrombin was dispersed were each prepared.
5. A knee femoral condyle of an experimental beagle was exposed, and a defect region having a diameter of about 6 mm and a depth of about 2 mm was formed in this part.
6. In order to manufacture a mold for scaffolding, 3D data of the defect cartilage site of the experimental beagle was obtained using a 3D scanner, and a medical grade polycaprolactone (PCL) external wall was printed using a 3D Bio 3D printer (INVIVO, ROKIT) based on this data. Furthermore, after the MCCM/SVF/fibrinogen solution and the calcium chloride solution in which thrombin was dispersed were each sequentially applied into the mold for scaffolding using a 3D Bio 3D printer (INVIVO, ROKIT), a customized scaffold for cartilage regeneration was manufactured by hardening the solutions.
7. 100 µl of a fibrin glue (Beriplast) was injected into the defect region of the experimental beagle, and the manufactured customized scaffold for cartilage regeneration was implanted, and then the surgical site was sutured.
8. After 6 weeks, it was confirmed whether chondrocytes were formed in the implanted customized scaffold for cartilage regeneration.

### Comparative Example 2

An animal experiment was performed in the same manner as described above by mixing only SVF with an aprotinin liquid in which fibrinogen was mixed without MCCM.

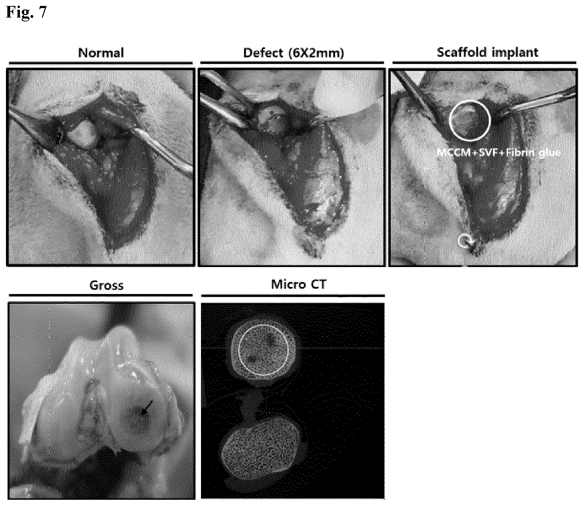

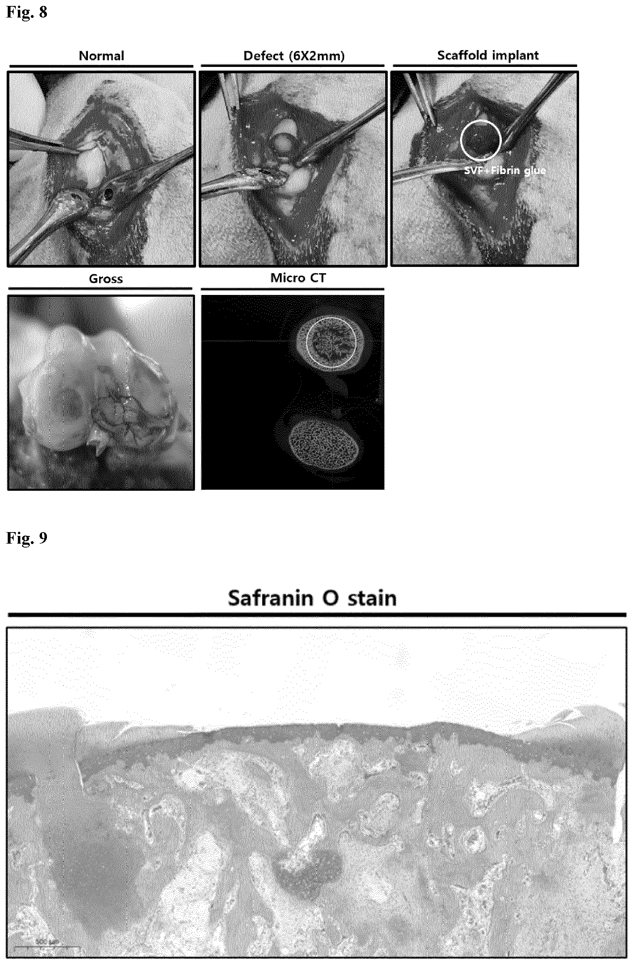

FIGS. 7 and 8 each show images during an animal experimental procedure of Example 4 and Comparative Example 2 according to Experimental Example 2 and a micro CT imaging image for a defect region 6 weeks after the implementation of a scaffold. In FIGS. 7 and 8, it can be confirmed that the micro CT imaging results according to Example 4 exhibit a very high bone density compared to the micro CT imaging results according to Comparative Example 2. Through this, it can be confirmed that in the case of Example 4 using MCCM, there is a high ability to produce the cartilage.

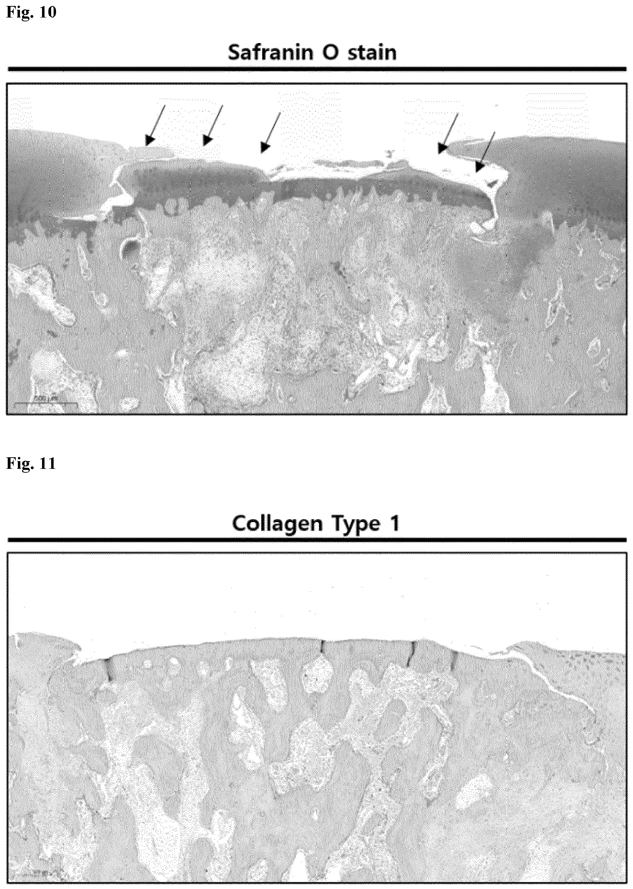

FIGS. 9 and 10 each show a result of performing safranin O staining on a defect region 6 weeks after the implantation of Comparative Example 2 and Example 4 according to Experimental Example 2. As a result of safranin O staining, it could be confirmed that only in the case of Example 4, the cartilage in the defect region was regenerated. This can be confirmed in a portion where a red-expressed volume is generated in the portion marked with an arrow in FIG 10. Furthermore, the red-expressed portion in FIG. 9 is a region where only normal cartilage remains when the defect region is manufactured.

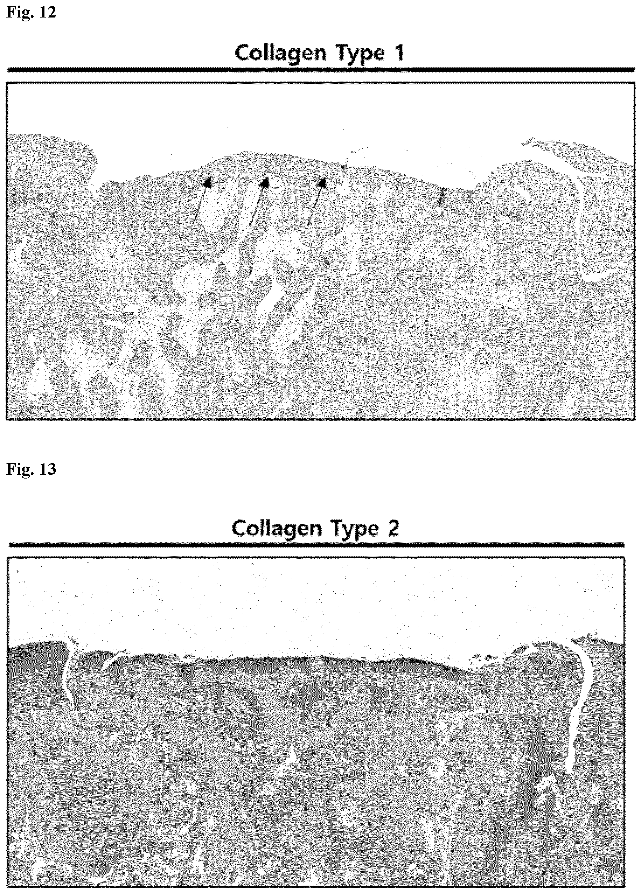

FIGS. 11 and 12 each show a result of performing an immunohistochemistry (IHC) treatment with collagen type 1 in order to confirm what type of cartilage is regenerated in a defect region 6 weeks after the implantation of Comparative Example 2 and Example 4 according to Experimental Example 2. The collagen type 1 binds to an antibody for confirming fibrocartilage, and serves to detect only fibrocartilage and express the fibrocartilage in brown. According to FIG. 12, it can be confirmed that the regenerated portion in the defect region is expressed in blue, and it can be seen that the regenerated cartilage is not fibrocartilage, but hyaline cartilage, and is regenerated into chondrocytes suitable for the defect region. In contrast, in the case of FIG. 11, a region differentiating into hyaline cartilage could not be confirmed.

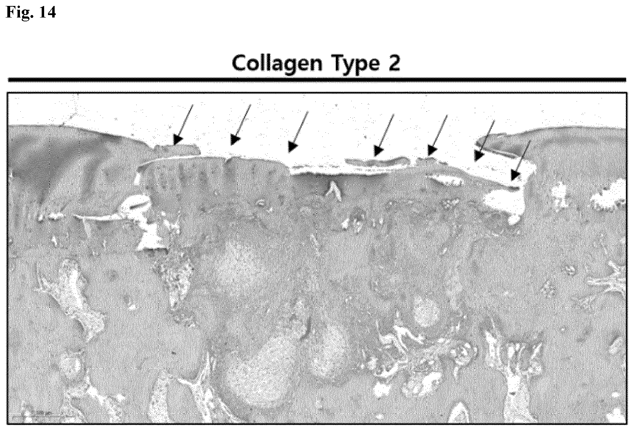

FIGS. 13 and 14 each show a result of performing an immunohistochemistry (IHC) treatment with collagen type 2 in order to confirm what type of cartilage is regenerated in a defect region 6 weeks after the implantation of Comparative Example 2 and Example 4 according to Experimental Example 2. The collagen type 2 binds to an antibody for confirming hyaline cartilage, and serves to detect only hyaline cartilage and express the hyaline cartilage in brown. According to FIG. 14, it can be confirmed that the regenerated portion in the defect region is expressed in brown, and it can be seen that the regenerated cartilage is not fibrocartilage, but hyaline cartilage, and is regenerated into chondrocytes suitable for the defect region. In contrast, in the case of FIG. 13, a region differentiating into hyaline cartilage could not be confirmed.

Through the results of the Examples and the Comparative Examples, it can be confirmed that when a customized scaffold for cartilage regeneration simultaneously using an adipose tissue-derived stromal vascular fraction and hyaline cartilage powder is implanted as in the Examples, the differentiation into chondrocytes is effectively performed, and furthermore, the diffusion and regeneration of chondrocytes are improved.

## Claims

1. A bioink composition for cartilage regeneration, the bioink composition comprising:
a first liquid comprising an adipose tissue-derived stromal vascular fraction, hyaline cartilage powder, and fibrinogen, wherein the first liquid comprises fibrinogen in an amount of 65 mg to 115 mg per ml of the first liquid; and
a second liquid comprising thrombin dissolved in a calcium chloride solution, wherein the second liquid comprises thrombin in an amount of 400 IU to 600 IU and 5 mg to 6.5 mg of calcium chloride per ml of the second liquid.

2. The bioink composition of claim 1, wherein a content of the adipose tissue-derived stromal vascular fraction is 10⁵ to 10⁷ cells per ml of the first liquid.

3. The bioink composition of claim 1, wherein a concentration of the hyaline cartilage powder is 0.005% (w/v) to 0.1% (w/v).

4. The bioink composition of claim 1, wherein the first liquid further comprises aprotinin.

5. The bioink composition of claim 1, wherein the adipose tissue-derived stromal vascular fraction is extracted from an autologous adipose tissue.

6. The bioink composition of claim 1, wherein the hyaline cartilage powder is derived from allogeneic hyaline cartilage.

7. The bioink composition of claim 1, wherein the hyaline cartilage powder has a particle diameter of 30 µm to 300 µm.

8. The bioink composition of claim 1, wherein the hyaline cartilage powder is derived from costal cartilage.

9. A method for manufacturing a customized scaffold for cartilage regeneration, the method comprising:
a) obtaining 3D data of a defect cartilage site using a 3D scanner, and manufacturing a mold for scaffolding using a 3D printer;
b) using a first liquid comprising an adipose tissue-derived stromal vascular fraction, hyaline cartilage powder, and fibrinogen, wherein the first liquid comprises fibrinogen in an amount of 65 mg to 115 mg per ml of the first liquid, and applying the first liquid in the mold for scaffolding to form a first layer;
c) applying a second liquid comprising thrombin dissolved in a calcium chloride solution onto the first layer to form a second layer, wherein the second liquid comprises thrombin in an amount of 400 IU to 600 IU and 5 mg to 6.5 mg of calcium chloride per ml of the second liquid; and
d) forming a customized scaffold for cartilage regeneration by allowing the first layer and the second layer to react with each other.

10. The method of claim 9, wherein steps b) and c) are alternately repeated.

11. The method of claim 9, wherein step d) is completed within 10 minutes.

12. The method of claim 9, wherein steps b) and c) are performed using inkjet printing or 3D printing.

13. A customized scaffold for cartilage regeneration manufactured using the method of claim 9.

## Patentansprüche

1. Eine Biotintenzusammensetzung für die Knorpelregeneration, wobei die Biotintenzusammensetzung umfasst:
eine erste Flüssigkeit, umfassend eine aus Fettgewebe stammende stromale Gefäßfraktion, hyalines Knorpelpulver und Fibrinogen, wobei die erste Flüssigkeit Fibrinogen in einer Menge von 65 mg bis 115 mg pro ml der ersten Flüssigkeit umfasst; und
eine zweite Flüssigkeit, umfassend in einer Calciumchloridlösung gelöstes Thrombin, wobei die zweite Flüssigkeit Thrombin in einer Menge von 400 IE bis 600 IE und 5 mg bis 6,5 mg Calciumchlorid pro ml der zweiten Flüssigkeit umfasst.

2. Die Biotintenzusammensetzung gemäß Anspruch 1, wobei der Gehalt der aus Fettgewebe stammenden stromalen Gefäßfraktion 10⁵ bis 10⁷ Zellen pro ml der ersten Flüssigkeit beträgt.

3. Die Biotintenzusammensetzung gemäß Anspruch 1, wobei die Konzentration des hyalinen Knorpelpulvers 0,005% (G/V) bis 0,1% (G/V) beträgt.

4. Die Biotintenzusammensetzung gemäß Anspruch 1, wobei die erste Flüssigkeit ferner Aprotinin enthält.

5. Die Biotintenzusammensetzung gemäß Anspruch 1, wobei die aus Fettgewebe stammende stromale Gefäßfraktion aus autologem Fettgewebe extrahiert wird.

6. Die Biotintenzusammensetzung gemäß Anspruch 1, wobei das hyaline Knorpelpulver aus allogenem hyalinem Knorpel gewonnen wird.

7. Die Biotintenzusammensetzung gemäß Anspruch 1, wobei das hyaline Knorpelpulver einen Teilchendurchmesser von 30 µm bis 300 µm aufweist.

8. Die Biotintenzusammensetzung gemäß Anspruch 1, wobei das hyaline Knorpelpulver aus Rippenknorpel gewonnen wird.

9. Ein Verfahren zur Herstellung eines kundenspezifischen Gerüsts für die Knorpelregeneration, wobei das Verfahren umfasst:
a) Gewinnen von 3D-Daten einer Knorpeldefektstelle mit einem 3D-Scanner und Herstellung einer Form zur Gerüstbildung mit einem 3D-Drucker;
b) Verwenden einer ersten Flüssigkeit, umfassend eine aus Fettgewebe stammende stromale vaskuläre Fraktion, hyalines Knorpelpulver und Fibrinogen, wobei die erste Flüssigkeit Fibrinogen in einer Menge von 65 mg bis 115 mg pro ml der ersten Flüssigkeit umfasst, und Aufbringen der ersten Flüssigkeit in der Form zur Gerüstbildung, um eine erste Schicht zu bilden;
c) Aufbringen einer zweiten Flüssigkeit, umfassend in einer Calciumchloridlösung gelöstes Thrombin, auf die erste Schicht, um eine zweite Schicht zu bilden, wobei die zweite Flüssigkeit Thrombin in einer Menge von 400 IE bis 600 IE und 5 mg bis 6,5 mg Calciumchlorid pro ml der zweiten Flüssigkeit enthält; und
d) Bilden eines kundenspezifischen Gerüsts für die Knorpelregeneration durch miteinander reagieren lassen der ersten Schicht und der zweiten Schicht.

10. Das Verfahren gemäß Anspruch 9, wobei die Schritte b) und c) abwechselnd wiederholt werden.

11. Das Verfahren gemäß Anspruch 9, wobei Schritt d) innerhalb von 10 Minuten abgeschlossen wird.

12. Das Verfahren gemäß Anspruch 9, wobei die Schritte b) und c) mittels Tintenstrahldruck oder 3D-Druck durchgeführt werden.

13. Ein kundenspezifisches Gerüst für die Knorpelregeneration, hergestellt unter Verwendung des Verfahrens nach Anspruch 9.

## Revendications

1. Composition de bioencre pour la régénération de cartilage, la composition de bioencre comprenant :
un premier liquide comprenant une fraction vasculaire stromale dérivée de tissu adipeux, de la poudre de cartilage hyalin, et du fibrinogène, dans laquelle le premier liquide comprend du fibrinogène en une quantité de 65 mg à 115 mg par ml du premier liquide ; et
un deuxième liquide comprenant de la thrombine dissoute dans une solution de chlorure de calcium, dans laquelle le deuxième liquide comprend de la thrombine en une quantité de 400 UI à 600 UI et 5 mg à 6,5 mg de chlorure de calcium par ml du deuxième liquide.

2. Composition de bioencre selon la revendication 1, dans laquelle la teneur en la fraction vasculaire stromale dérivée de tissu adipeux est de 10⁵ à 10⁷ cellules par ml du premier liquide.

3. Composition de bioencre selon la revendication 1, dans laquelle la concentration de la poudre de cartilage hyalin est de 0,005 % (p/v) à 0,1 % (p/v).

4. Composition de bioencre selon la revendication 1, dans laquelle le premier liquide comprend en outre de l'aprotinine.

5. Composition de bioencre selon la revendication 1, dans laquelle la fraction vasculaire stromale dérivée de tissu adipeux est extraite d'un tissu adipeux autologue.

6. Composition de bioencre selon la revendication 1, dans laquelle la poudre de cartilage hyalin dérive de cartilage hyalin allogénique.

7. Composition de bioencre selon la revendication 1, dans laquelle la poudre de cartilage hyalin a un diamètre de particule de 30 µm à 300 µm.

8. Composition de bioencre selon la revendication 1, dans laquelle la poudre de cartilage hyalin dérive de cartilage costal.

9. Procédé pour fabriquer un échafaudage personnalisé pour la régénération de cartilage, le procédé comprenant :
a) l'obtention de données 3D d'un site de cartilage défectueux utilisant un scanneur 3D, et la fabrication d'un moule pour échafaudage utilisant une imprimante 3D ;
b) l'utilisation d'un premier liquide comprenant une fraction vasculaire stromale dérivée de tissu adipeux, d'une poudre de cartilage hyalin, et du fibrinogène, dans lequel le premier liquide comprend du fibrinogène en une quantité de 65 mg à 115 mg par ml du premier liquide, et l'application du premier liquide dans le moule pour échafaudage afin de former une première couche ;
c) l'application d'un deuxième liquide comprenant de la thrombine dissoute dans une solution de chlorure de calcium sur la première couche afin de former une deuxième couche, dans lequel le deuxième liquide comprend de la thrombine en une quantité de 400 UI à 600 UI et 5 mg à 6,5 mg de chlorure de calcium par ml du deuxième liquide ; et
d) la formation d'un échafaudage personnalisé pour la régénération de cartilage par l'opération consistant à laisser la première couche et la deuxième couche réagir l'une avec l'autre.

10. Procédé selon la revendication 9, dans lequel les étapes b) et c) sont répétées en alternance.

11. Procédé selon la revendication 9, dans lequel l'étape d) est complétée en 10 minutes.

12. Procédé selon la revendication 9, dans lequel les étapes b) et c) sont effectuées par utilisation d'une impression par jet d'encre ou d'une impression 3D.

13. Echafaudage personnalisé pour la régénération de cartilage fabriqué par utilisation du procédé de la revendication 9.
